# EUROPEAN PATENT SPECIFICATION

(11) **EP 1 666 093 B1**
(45) Date of publication and mention of the grant of the patent: **21.01.2009**
(21) Application number: 04027495.3
(22) Date of filing: 19.11.2004
(51) Int. Cl.: A61Q 5/06, A61K 8/04, A61K 8/81

(54) **Aerosol hair styling composition comprising amphoteric and non-ionic polymers**
Aerosolprodukt zur Haarbehandlung enthaltend amphoterische sowie nichtionische Polymere
Composition de coiffage sous forme aérosol comprenant des polymères amphotériques et non-ioniques

(43) Date of publication of application: 07.06.2006
(73) Proprietor: KPSS-Kao Professional Salon Services GmbH, 64297 Darmstadt (DE)
(72) Inventor: Cajan, Christine, 56130 Bad Ems (DE); Weichaus, Dirk, 64404 Bickenbach (DE)

(56) References cited:
- EP-A- 0 370 764
- EP-A- 0 919 219
- WO-A-92/07545
- WO-A-99/13843
- DE-A1- 4 315 405
- DE-A1- 10 216 498
- NATIONAL STARCH - CHEMICAL, 16 September 2004 (2004-09-16), XP002321348 PERFORMANCE MATCHES FOR EUROPEAN MOUSSES USING CELQUAT L200 AND AMAZE Retrieved from the Internet: URL:http://www.personalcarepolymers.com/Do c/Rpt/mousses.pdf> [retrieved on 2005-03-15]
- PATENT ABSTRACTS OF JAPAN vol. 015, no. 238 (C-0841), 19 June 1991 (1991-06-19) -& JP 03 074319 A (SUNSTAR INC), 28 March 1991 (1991-03-28)
- NATIONAL SARCH PERSONAL CARE, 12 January 2004 (2004-01-12), XP002321349 AMPHOMER HARD HOLDING HAIR FIXATIVE Retrieved from the Internet: URL:http://www.personalcarepolymers.com/Do c/EN/TSB/0284910.pdf> [retrieved on 2005-03-14]

## Description

The present invention is related to a sprayable aerosol foam styling composition for hair, especially for application to hair root area.

Hair styling compositions have been known in the literature for many years. Foam styling products are applied to the whole body of hair either wet or dry usually by hand.

In many consumer research activities it has especially been found out that consumers having fine hair are mostly priotorising the volume as the main hair related problem after washing and conditioning hair. For those, it is customary to use styling product to achieve volume up effect by fixing the hair in certain shape using conventional hair styling products. This brings about problems in hair feeling, especially when touching hair does not feel soft and natural as hair becomes hard and hair surface becomes rough by coverage with fixing polymers.

Another styling foams related point is the relatively long stability of the dispensed foam. Long stability of foam does not allow working out of the product into hair appropriately. Therefore, in practice usually relatively instable foams are desired. On the other hand, foam is preferred form of application as it makes observation of applied amount and areas possible as well as prevents unwanted product spreading all over hair. In other words, foam helps to carry out successful application of styling product. The desire is there for a product with matched foaming and foam stability performance.

Partial application of styling products is often carried out in usual hair dressing practice. The reason for this is that the desired effect is partial i.e. settinghair only at forehead area, or that the hair feels so unnatural and rough after application of styling composition that consumers want to prevent such feeling on whole head.

By application of a foam type of styling composition in the form of a spray, it is possible to carry out partial applications so that the product is only applied at the parts where it is needed/desired. Especially the compositions designed for achieving a volume up effect are the best candidates for such kind of application as by their partial application especially at the hair root area the optimum effect can be obtained.

Anionic polymers are used usually to achieve long lasting volume up effect. Although their preference, they make the hair extremely rough and because of this especially in the wet hair effecting further processing in hair styling. Nonionic and cationic polymers, contrary to the anionic polymers, are easy to apply and do not result in extremely rough hair feeling, but their high sensitivity to humidity makes achieving long lasting volume effect nearly impossible.

The present invention starts from the problem outlined above and aims at developing a foaming spray styling product with excellent volume up effect and with quick breaking foam, which allows partial application especially at hair root area. Furthermore, the objective of the present invention is as well to develop sprayable foaming hair styling composition, which does not make hair extremely rough and hard and therefore easy to work out into hair during styling.

The inventors have surprisingly found out that an organic solvent free (other than propellants) hair styling composition wherein organic solvent concentration is less than 5% by weight of the liquid composition when row materials are used dissolved in organic solvents, comprising at least one amphoteric polymer and at least one non-ionic polymer selected from vinylpyrrolidone/vinylacetate copolymer, polyvinylpyrrolidone, vinylpyrrolidone/vinylacetate/ vinylpropionate copolymer wherein at least one non-ionic polymer and at least one amphoteric polymer are comprised at a ratio between 1:3 and 1:15, by weight, in an aqueous medium pressurised with a propellant mixture of C₂-C₄ alkanes /andor haloalkanes or their mixtures and dimethylether solves the above-mentioned problems.

The styling composition of the present invention is organic solvent free, other than propellants. It should be noted that low concentration of organic solvents, i.e. less than 5% by weight of the liquid composition, may be contained in the compositions when using raw materials dissolved in organic solvents.

EP 1 205 174 B1 discloses hair treatment compositions for achieving volume up effect comprising polymer with strong setting effect, foam breaking agent, surfactant, at least 60% water and at least one propellant. The quick beraking foam is achieved by addition of foam breaking agents such as alcohol (organic solvent at a concentration higher than 10% by weight used in the examples), oils and silicone oils.

EP 663203 B1 discloses cosmetic compositions in an aerosol container comprising at least one anionic and/or cationic foaming polymer wherein the composition foams spontaneously when coming in contact with a surface especially hair surface. The document is silent on any use of amphoteric polymers in such compositions and furthermore presence of organic solvents is not excluded at all.

EP 370 764 A2 discloses hair styling mousse composition comprising non ionic polymer and anionic polymer in an aqueous medium. However, the ratio of the nonionic and amphoteric polymers is different.

As the compositions of the present invention is free of organic solvents the compositions comprise at least 80% preferably 85% and more preferably 90% by weight water calculated to total composition excluding propellant gas.

Amphoteric or zwitterionic polymers suitable for the compositions of the present inventions are for example copolymerisate of n-octylacrylamide, acrylic or metahcrylic acid and tert.-butylaminoethylmethacrylate known with its trade name Amphomer, copolymer of methacryloylethylbetaine and alkyl methacrylate known as Yukaformer.

Among those the preferred amphoteric polymer is copolymers of N-octyl acrylamide, (meth)acrylic acid and tert.-butyl aminoethyl methacrylate of the type "Amphomer®".

Amphoteric polymers are comprised in the compositions of the present invention at a concentration of 0.5 to 10%, preferably 0.5 to 7.5%, more preferably 0.5 to 5% and most preferably 1 to 5% by weight calculated to total composition excluding propellant gas.

Nonionic polymers suitable are first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, VA 73 from BASF AG.

Further non-ionic polymer suitable for compositions of the present invention is vinylpyrrolidone/vinylacetae/vinylpropionate copolymer known with the trade name Luviskol VAP 343 as well from BASF.

Natural non-ionic polymers are as well suitable for the composition of the present invention. Those are such as cellulose, chitosan, guar gum, neutralised shellac and their derivatives.

Nonionic polymers are comprised in the compositions of the present invention at a concentration of 0.05 to 2%, preferably 0.05to 1.5%, more preferably 0.1 to 1% and most preferably 0.1 to 0.75% by weight calculated to total composition excluding propellant gas.

pH of the composition is preferably around neutral pH, more preferably between 6.5 to 9, most preferably between 7 to 8.5.

It should be noted that in the compositions of the present invention non-ionic polymers and amphoteric polymers are contained at a ratio 1:3 to 1:15, preferably 1:3 to 1:10 and most preferably 1:4 to 1:8 by weight.

As the propellant gas mixture, the composition of the present invention comprises C2-C4 alkanes and/or haloalkanes or their mixtures and dimethylether. As alkanes preferred are n-butane, isobutene, Propan butane 1,1-difluoroethane and (F152a) tetrafluoroethane (F134) or their mixtures. The propellant concentration in the aerosol product is in the range of 2 to 30%, preferably 5 to 25% and more preferably 10 to 25% by weight calculated to total composition. The ratio of alkane or their mixture with haloalkanes to dimethylether is in the range of 1:2 to 1:5, preferably 1:2 to 1:4, by weight. In the case that only haloalkanes such as difluoureethane and dimethylether mixture is used as propellant gas the ratio of haloalkanes to dimethylether is between 1:1 to 5:1, preferably 1:1 to 4:1.

Styling composition of the present invention may comprise additional components such as surfactants of non-ionic, cationic, amphoteric and/or zwitterionic character. The non-ionic surfactants are used in the compositions of the present invention especially as solubilizers when incorporating water insoluble and/or oily compounds, especially of fragrance compounds into the composition. Those ethoxylates of hydrogenated castor oil such as PEG 40 hydrogenated castor oil are found to be particularly suitable know with the trade name Cremophor from BASF.

Other non-ionic surfactants to mention are alkyl polyglucosides of the general formula

R₁-O-(R₂O)ₙ-Zₓ,

wherein R₁ is an alkyl group with 8 to 18 carbon atoms, R₂ is an ethylene or propylene group, Z is a saccharide group with 5 to 6 carbon atoms, n is a number from 0 to 10 and x is a number between 1 and 5.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R"}, as well as fatty alcohol ethoxylates.

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 25, preferably about 10 and about 20.

As further surfactant component, the compositions according to the invention may comprise amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Hair styling composition may contain further cationic cationic conditioning ingredients according to the formula 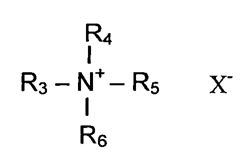 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 1 - 4 or

R₈ CO O (CH₂)ₙ

where R₈ is saturated or unsaturated, branched or non-branched alkyl chain with 7-21 C atoms and n has typical value of 1 - 4, and
R₄ is H or unsaturated or saturated, branched or non-branched alkyl chain with 1 - 22 C atoms or

R₇ CO NH (CH₂)ₙ

or

R₈ CO O (CH₂)ₙ

where R₇, R₈ and n are same as above.

R₅ and R₆ are H or lower alkyl chain with 1 to 4 Carbon atoms, and X is typically chloride, bromide or methosulfate.

Typical examples of those ingredients are cetyltrimethly ammonium chloride, steartrimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate. Those can at the same time serve as solubilizing agents for those ingredients difficult to integrate into the formulations.

Concentration of surfactants of non-ionic, cationic, amphoteric and/or zwitterionic character is usually low, in any case lower than around 2%, preferably in the range of 0.05 to 1.5% and more preferably 0.1 to 1% and most preferably 0.01 to 0.75% by weight calculated to total composition excluding propellants.

It is the subject matter of the present invention that the hair styling composition contains 2% Octylacrylamide/Acrylates/Butylaminoethyl Methacrylate Copolymer, 0.25% VP/VA Copolymer, 0.15% cetrimoniumchloride, 0.20% PEG-40 hydrogenated castor oil, and 0.20% fragrance (all values are by weight) in water without any added organic solvent and at pH 8.5 adjusted with appropriate amount of aminomethylpropanol. The composition is further filled into an aerosol can with 80% aqueous composition as above with 4% by weight n-butane and 16% by weight dimethylether as propellants.

It is still further subject matter of the present invention that the hair styling composition contains 2% Octylacrylamide/Acrylates/Butylaminoethyl Methacrylate Copolymer, 0.25% PVP, 0.15% cetrimoniumchloride, 0.20% PEG-40 Hydrogenated castor oil, and 0.20% fragrance (all values are by weight) in water without any added organic solvent and at pH 8.5 adjusted with appropriate amount of aminomethylpropanol. The composition is further filled into an aerosol can with 80% aqueous composition as above with 5% by weight isobutane and 15% by weight dimethylether as propellants.

The composition of the present invention may comprise additional compounds customarily found in hair styling compositions especially those of packed or confectioned as an aerosol product. Some of them to mention are pH adjusting agents, especially aminomethylpropanol, fragrances, preservatives, and UV filters.

Furthermore, a volume up effect is achieved on head of a person according to the present invention in a process wherein composition according to the present invention comprising at least one amphoteric polymer, at least one nonionic polymer in an aqueous medium pressurised with a propellant mixture of C2-C4 alkanes /andor haloalkanes or their mixtures and dimethylether is applied onto wet/dry hair.

Additionally, in the process as defined in the paragraph above, before application of the styling composition to hair hair is washed with a cleansing composition and eventually conditioned with a conditione, preferably of rinse off.

The following examples are to illustrate the invention.

### Example 1

| | | |
|---|---|---|
| Octylacrylamide/Acrylates/ | | 2.00 % by weight |
| Butylaminoethyl Methacrylate Copolymer | | |
| VP/VA Copolymer | | 0.25 |
| Cetrimoniumchloride | | 0.15 |
| Fragrance | | 0.20 |
| PEG-40 Hydrogenated castor oil | | 0.20 |
| Aminomethylpropanol | | q.s. pH 8.5 |
| Water | | add 100 % |

| Filling ratio: | |
|---|---|
| Bulk | 80 % by weight |
| n-Butane | 4 % |
| Dimethylether | 16 % |

### Example 2

| | | |
|---|---|---|
| Octylacrylamide/Acrylates/ | | 2.00 % by weight |
| Butylaminoethyl Methacrylate Copolymer | | |
| PVP | | 0.25 |
| Cetrimoniumchloride | | 0.15 |
| Fragrance | | 0.20 |
| PEG-40 Hydrogenated castor oil | | 0.20 |
| Aminomethyl propanol | | q.s. pH 8.5 |
| Water | | add 100 |

| Filling ratio: | |
|---|---|
| Bulk | 80 % by weight |
| Isobutane | 5 % |
| Dimethylether | 15 % |

### Example 3

| | | |
|---|---|---|
| Octylacrylamide/Acrylates/ | | 2.50 % by weight |
| Butylaminoethyl Methacrylate Copolymer | | |
| VPNA Copolymer | | 0.50 |
| Cetrimoniumchloride | | 0.15 |
| Fragrance | | 0.20 |
| PEG-40 Hydrogenated castor oil | | 0.20 |
| Aminomethylpropanol | | q.s. pH 8.5 |
| Water | | add 100 % |

| Filling Ratio: | |
|---|---|
| Bulk | 80 % |
| Difluoroethane | 14 % |
| Dimethylether | 6 % |

### Example 4

| | | |
|---|---|---|
| Octylacrylamide/Acrylates/ | | 2.00 % by weight |
| Butylaminoethyl Methacrylate Copolymer | | |
| VPNA Copolymer | | 0.25 |
| Cetrimoniumchloride | | 0.15 |
| Fragrance | | 0.20 |
| PEG-40 Hydrogenated castor oil | | 0.20 |
| Aminomethylpropanol | | q.s. pH 8.5 |
| Water | | add 100 % |

| Filling Ratio: | |
|---|---|
| Bulk | 80 % by weight |
| n-Butane | 4 % |
| Dimethylether | 16 % |

### Example 5

| | | |
|---|---|---|
| Octylacrylamide/Acrylates/ | | 1.00 % by weight |
| Butylaminoethyl Methacrylate Copolymer | | |
| Aminoxide Methacrylate Copolymer | | 1.00 |
| VP/VA Copolymer | | 0.50 |
| Cetrimoniumchloride | | 0.15 |
| Fragrance | | 0.20 |
| PEG-40 Hydrogenated castor oil | | 0.20 |
| Aminomethylpropanol | | q.s. pH 8.5 |
| Water | | add 100 % |

| Filling Ratio: | |
|---|---|
| Bulk | 80 % by weight |
| n-Butane | 4 % |
| Dimethylether | 16 % |

The following examples are prepared for comparative purposes

### Comparative example 1

| | |
|---|---|
| Ethanol | 10.0 g |
| Aquaflex SF-64 (ISP) | 5.6 g |
| (Isobutylene/Ethylmaleimide/Hydroxyethylmaleimide Copolymer. 40 % in 27% water/33% ethanol) | |
| Cetyltrimethylammoniumchlorid | 0.15 g |
| Laureth-4 | 0.20 g |
| Parfüm | 0.20 g |
| Ethanol | 10.0 g |
| Water | add 100g |

| | |
|---|---|
| Bulk | 80 % |
| n-Butan | 4% |
| DME | 16 % |

### Comparative example 2

| | |
|---|---|
| Methylvinylether/Butylmonomaleat-Copolymer | 4.00 % by weight |
| (Gantrez ES 425 50 % ethanolic solution) | |
| Aminomethylpropanol | q.s. pH 8.5 |
| Parfume, preservative | q.s. |
| Water | add 100 % |

| Filling: | |
|---|---|
| Bulk | 70 % by weight |
| Isobutan | 10 % |
| Dimethylether | 20 % |

### Comparative example 3

| | |
|---|---|
| Copolymer aus Acrylsäure. Ethylacrylat und N-tertiär-Butylacrylamid | 2.00 % by weight |
| Parfume, preservative | q.s. |
| Aminomethylpropanol | q.s. pH 8.0 |
| Water | add 100 % |

| Filling: | |
|---|---|
| Bulk | 75% |
| Isobutan | 8 % |
| DME | 17 % |

### Comparative example 4

| | |
|---|---|
| Polyquaternium-46 | 1.00 % by weight |
| VP/VA Copolymer | 1.50 |
| Cetrimoniumchlorid | 0.15 |
| Parfume, preservative | q.s. |
| Water | add 100 |

| Filling: | |
|---|---|
| Bulk | 80 % |
| n-Butan | 8% |
| DME | 12 % |

### Comparative test

In the comparative tests, performance of each comparative example as given above is compared to the example of the invention.

The products are tested in an half side test on 10 female aging 20 to 45 and having ear to shoulder length hair. Test person's hair is washed with a shampoo composition designed for normal hair (shampoo for normal hair taken from the brand Goldwell available commercially) and to each half side 3 g of product is sprayed at the root area and spreaded by hand and the hair is dried with a hair drier using a hair brush. Afterwards, 3 hairdressers evaluated hair properties independently and for each test an arithmetic mean of the 3 is calculated. The evaluation scale was as follows:

| | |
|---|---|
| 0 = | does not exisit |
| 1 = | very low level of effect |
| 2 = | low level of effect |
| 3 = | effect exists |
| 4 = | strong effect |
| 5 = | very strong effect |

Only the evaluation of volume was repeated after 24 h again in order to show achieved long lasting volume up effect.

The total score for each side is than calculated as sum of the all individual tests. Therefore for each point evaluated the maximum score is 50 and the minimum is 0. The higher the number in the tables below, the better the effect.

**Table I: Test 1 comparative example 1 versus example 1**

| Perfomance criteria | Example 1 | Comparative example 1 |
|---|---|---|
| Blow drying ability | 44 | 40 |
| Flexability | 45 | 33 |
| Elasticity | 43 | 15 |
| Nice hair feel | 41 | 45 |
| Volume | 48 | 15 |
| Volume after 24 h | 45 | 12 |

**Table II: Test 2 comparative example 2 versus example 1**

| Perfomance criteria | Example 1 | Comparative example 2 |
|---|---|---|
| Blow drying ability | 45 | 25 |
| Flexability | 41 | 25 |
| Elasticity | 45 | 19 |
| Nice hair feel | 43 | 44 |
| Volume | 46 | 12 |
| Volume after 24 h | 41 | 12 |

**Table III: Test 3 comparative example 3 versus example 1**

| Perfomance criteria | Example 1 | Comparative example 3 |
|---|---|---|
| Blow drying ability | 43 | 26 |
| Flexability | 44 | 18 |
| Elasticity | 48 | 15 |
| Nice hair feel | 42 | 20 |
| Volume | 48 | 30 |
| Volume after 24 h | 44 | 16 |

**Table IV: Test 4 comparative example 4 versus example 1**

| Perfomance criteria | Example 1 | Comparative example 4 |
|---|---|---|
| Blow drying ability | 44 | 43 |
| Flexability | 44 | 18 |
| Elasticity | 48 | 15 |
| Nice hair feel | 42 | 20 |
| Volume | 48 | 30 |
| Volumn after 24h | 44 | 16 |

From the above test it is clear that in all comparative tests the inventive composition 1 of the present invention showed superior performance in most of the performance criteria. Especially worth to mention is the long lasting volume up effect. In all tests the composition of the present invention showed superior performance to those of comparative examples.

## Claims

1. Styling composition for hair **characterised in that** it is free of organic solvents other than propellants wherein organic solvent concentration is less than 5% by weight of the liquid composition when raw materials are used dissolved in organic solvents and comprises at least one amphoteric polymer, and at least one non-ionic polymer selected from vinylpyrrolidone/vinylacetate copolymer, polyvinylpyrrolidone, vinylpyrrolidone/vinylacetate/vinylpropionate copolymer wherein at least one nonionic polymer and at least one amphoteric polymer are comprised at a ratio between 1:3 and 1:15, by weight, in an aqueous medium and pressurised with a propellant mixture of C₂ - C₄ alkanes and/or haloalkanes or their mixtures with dimethylether.

2. Composition according to claim 1 **characterised in that** the amphoteric polymer is copolymer of N-octyl acrylamide, (meth)acrylic acid and tert.-butyl-aminoethyl methacrylate and comprised in the composition at a concentration of 0.5 to 10% by weight calculated to total composition excluding propellant gas.

3. Composition according to claims 1 and 2 **characterised in that** nonionic polymer is comprised in the composition at a concentration of 0.05 to 2% by weight calculated to total composition excluding propellant gas.

4. Composition according to any of the preceding claims **characterised in that** it comprises at least 80% water by weight calculated to total composition excluding propellant gas.

5. Composition according to any of the preceding claims **characterised in that** it comprises propellant gas mixture at a concentration of 2 to 30% by weight calculated to total composition and at a weight ratio of C₂ - C₄ alkanes and/or haloalkanes or their mixtures and dimethylether between 1:2 to 1:5.

6. Composition according to any of the preceding claims **characterised in that** it comprises additionally at least one surfactant selected from non-ionic, cationic, amphoteric and zwitterionic ones and at a concentration lower than 2% by weight, calculated to total composition excluding propellant gas.

7. Composition according to any of the preceding claims **characterised in that** it has a pH between 6.5 and 9.

8. Use of composition according to any of the preceding claims for increasing hair volume on head of a person.

9. Process for increasing volume of hair on head of a person **characterised in that** a composition according to claims 1 to 7 is applied onto wet and/or dry hair.

10. Process for increasing volume of hair on the head of a person **characterised in that** the hair is washed with a cleansing composition, eventually treated with a conditioner composition, preferably of a rinse off, and towel dried before application of a composition according to claims 1 to 7.

## Patentansprüche

1. Styling- Zusammensetzung für Haare, **dadurch gekennzeichnet, dass** sie von organischen Lösungsmittel, außer solchen wie Treibmitteln frei ist, - wobei die organische Lösungsmittelmenge kleiner als 5 Gew.- % der flüssigen Zusammensetzung ist, wenn in organischen Lösungsmitteln gelöste Rohstoffe verwendet werden -, und mindestens ein amphoterisches Polymer und mindestens ein nichtionisches Polymer, ausgewählt aus Vinylpyrrolidone/vinyl-acetate Copolymer, Polyvinylpyrrolidone, Vinylpyrrolidone/vinylacetate/vinyl-propionate Copolymer enthält, wobei mindestens ein nichtionisches Polymer und mindestens ein amphoterisches Polymer in einem Gewichtsverhältnis zwischen 1:3 und 1:5 in einem wässrigen Medium enthalten sind und mit einem Treibmittelgemisch von C₂ - C₄ Alkanen und/oder Haloalkanen oder deren Mischungen mit Dimethylether Druckgasverpackt werden.

2. Zusammensetzung nach Anspruch 1, **dadurch gekennzeichnet, dass** das amphoterische Polymer ein Copolymer von N-Cctyl acrylamide, (Meth)acrylic acid und tert.-Butyl aminoethyl methacrylate ist und in der Zusammensetzung in einer Menge von 0,5 bis 10 Gew.- %, berechnet auf die Gesamtzusammensetzung, ausgenommen Treibmittelgas, enthalten ist.

3. Zusammensetzung nach den Ansprüchen 1 und 2, **dadurch gekennzeichnet, dass** das nichtionische Polymer in der Zusammensetzung in einer Menge von 0,5 bis 2 Gew.- %, berechnet auf die Gesamtzusammensetzung, ausgenommen Treibmittelgas, enthalten ist.

4. Zusammensetzung nach einem der vorhergehenden Ansprüche, **dadurch gekennzeichnet, dass** sie mindestens 80 Gew.- % Wasser, berechnet auf die Gesamtzusammensetzung, ausgenommen Treibmittelgas, enthält.

5. Zusammensetzung nach einem der vorhergehenden Ansprüche, **dadurch gekennzeichnet, dass** sie eine Treibmittelgas- Mischung in einer Menge von 2 bis 30 Gew.- %, berechnet auf die Gesamtzusammensetzung, enthält, die in einem Gewichtsverhältnis von C₂ - C₄ Alkanen und/oder Haloalkanen oder deren Mischungen und Dimethylether zwischen 1:2 bis 1:5 vorliegt.

6. Zusammensetzung nach einem der vorhergehenden Ansprüche, **dadurch gekennzeichnet, dass** sie zusätzlich mindestens ein Tensid, ausgewählt aus nichtionischen, kationischen, amphoterischen und zwitterionischen Tensiden in einer Menge von weniger als 2 Gew.- %, berechnet auf die Gesamtzusammensetzung, ausgenommen Treibmittelgas, enthält.

7. Zusammensetzung nach einem der vorhergehenden Ansprüche, **dadurch gekennzeichnet, dass** sie einen pH- Wert zwischen 6,5 und 9 hat.

8. Verwendung der Zusammensetzung nach einem der vorhergehenden Ansprüche zur Erhöhung des Haarvolumens am Kopf einer Person.

9. Verfahren zur Erhöhung des Haarvolumens am Kopf einer Person, **dadurch gekennzeichnet, dass** eine Zusammensetzung nach den Ansprüchen 1 bis 7 auf feuchtes und/oder trockenes Haar aufgetragen wird.

10. Verfahren zur Erhöhung des Haarvolumens am Kopf einer Person, **dadurch gekennzeichnet, dass** auf das handtuchtrockene Haar, das vorher mit einem Reinigungsmittel gewaschen, und eventuell mit einem Konditionierungsmittel, vorzugsweise einem Rinse off behandelt wird, eine Zusammensetzung entsprechend der Ansprüche 1 bis 7 aufgetragen wird.

## Revendications

1. Composition coiffante pour cheveux **caractérisée en ce qu'**elle est exempte de solvants organiques autres que des agents propulsifs, dans laquelle la concentration en solvant organique est inférieure à 5 % en poids de la composition liquide lorsque l'on utilise des matières premières dissoutes dans des solvants organiques et qu'elle comprend au moins un polymère amphotère et au moins un polymère non ionique, choisis parmi un copolymère vinylpyrrolidone/acétate de vinyle, de la polyvinylpyrrolidone, un copolymère vinylpyrrolidone/acétate de vinyle/propionate de vinyle où au moins un polymère non ionique et au moins un polymère amphotère sont compris à un rapport situé entre 1:3 et 1:5, en poids, dans un milieu aqueux et mis sous pression avec un mélange propulseur d'alcanes C₂-C₄ et/ou d'haloalcanes ou leurs mélanges avec de l'éther diméthylique.

2. Composition selon la revendication 1, **caractérisée en ce que** le polymère amphotère est un copolymère de N-octylacrylamide, d'acide (méth)acrylique et de méthacrylate de tert-butylaminoéthyle et est compris dans la composition à une concentration de 0,5 à 10 % en poids calculée par rapport à la composition totale en excluant le gaz propulseur.

3. Composition selon les revendications 1 et 2, **caractérisée en ce que** le polymère non ionique est compris dans la composition à une concentration de 0,05 à 2 % en poids calculée par rapport à la composition totale en excluant le gaz propulseur.

4. Composition selon l'une quelconque des revendications précédentes, **caractérisée en ce qu'**elle comprend au moins 80 % en poids d'eau calculés par rapport à la composition totale en excluant le gaz propulseur.

5. Composition selon l'une quelconque des revendications précédentes, **caractérisée en ce qu'**elle comprend un mélange de gaz propulseurs à une concentration de 2 à 30 % en poids calculée par rapport à la composition totale et à un rapport en poids des alcanes C₂-C₄ et/ou des haloalcanes ou leurs mélanges et de l'éther diméthylique situé entre 1:2 et 1:5.

6. Composition selon l'une quelconque des revendications précédentes, **caractérisée en ce qu'**elle comprend en outre au moins un tensioactif choisi parmi les tensioactifs non ioniques, cationiques, amphotères et zwitterioniques et à une concentration inférieure à 2 % en poids, calculée par rapport à la composition totale en excluant le gaz propulseur.

7. Composition selon l'une quelconque des revendications précédentes, **caractérisée en ce qu'**elle possède un pH compris entre 6,5 et 9.

8. Utilisation d'une composition selon l'une quelconque des revendications précédentes, pour augmenter le volume des cheveux sur la tête d'une personne.

9. Procédé destiné à augmenter le volume des cheveux sur la tête d'une personne, **caractérisé en ce que** l'on applique une composition selon les revendications 1 à 7 sur des cheveux mouillés et/ou secs.

10. Procédé destiné à augmenter le volume des cheveux sur la tête d'une personne, **caractérisé en ce que** les cheveux sont lavés avec une composition nettoyante, traités éventuellement avec une composition de démélage des cheveux, de préférence éliminable par rinçage, et séchés à la serviette avant l'application d'une composition selon les revendications 1 à 7.
